# FASCICULE DE BREVET EUROPEEN

(11) **EP 2 379 484 B1**
(45) Date de publication et mention de la délivrance du brevet: **29.05.2013**
(21) Numéro de dépôt: 09797045.3
(22) Date de dépôt: 21.12.2009
(51) Int. Cl.: C07C 69/587, A61K 8/37, A61Q 9/00, A61K 31/22, A61P 17/10, A61P 17/08

(54) **ESTER DE DIOL ET D'ACIDE GRAS POLYINSATURE COMME AGENT ANTI-ACNE**
ESTER AUS MEHRFACH UNGESÄTTIGTER FETTSÄURE UND DIOL ALS ANTI-AKNEMITTEL
POLYUNSATURATED FATTY ACID AND DIOL ESTER AS AN ANTI-ACNE AGENT

(30) Priorité: 22.12.2008 FR 0858967
(43) Date de publication de la demande: 26.10.2011
(73) Titulaire: Pierre Fabre Dermo-Cosmétique, 92100 Boulogne-Billancourt (FR)
(72) Inventeur: REDOULES, Daniel, 31300 Toulouse (FR); DAUNES-MARION, Sylvie, 31000 Toulouse (FR); ARIES, Marie-Françoise, 31750 Escalquens (FR)
(74) Mandataire: Regimbeau
(86) Numéro de dépôt international: PCT/EP2009/067701
(87) Numéro de publication internationale: WO 2010/072738

(56) Documents cités:
- EP-A- 1 902 632
- WO-A-01/38288
- WO-A-98/13330
- WO-A-2006/019186

## Description

La présente invention concerne des esters d'alcane diol et d'acide gras polyinsaturé, plus particulièrement les omégas 3 et les omégas 6, ainsi que les compositions pharmaceutiques et cosmétiques les contenant, leur procédé de préparation et leur utilisation, notamment dans le traitement de l'acné ou de la dermite séborrhéique.

Les alcanes diol sont des composés utilisés dans de nombreux domaines tels que la cosmétologie ou l'agroalimentaire. On peut en particulier citer leurs utilisations en tant que conservateur grâce à leurs propriétés bactériostatiques. Ainsi, les alcanes diol constituent un moyen de lutte contre les colonisations fongiques et bactériennes et contribuent à une protection de nombreux produits cosmétiques ou agroalimentaires (Faergemann J, Fredriksson T. Sabouraudia : 1980 ; 18, 287-293). Ces diols présentent un large spectre d'activité et sont notamment efficaces vis-à-vis des espèces fongiques et bactériennes de type Gram + (Harb NA, Toama MA. Drug Cosmet Ind : 1976 ; 118, 40). En outre, la quasi absence de résistance acquise chez les micro-organismes permet aux alcanes diol d'être des partenaires importants dans l'élaboration de stratégies anti-résistance, notamment vis-à-vis du staphylocoque doré (Faergemann J, Hedner T, Larsson P : 2005 ; 85, 203-205 ; WO 2004/112765). Enfin leur très bonne tolérance permet une utilisation fréquente et à des doses qui dépassent plusieurs pour cent.

En particulier, les alcanes diol 1,2 ont des activités bactériostatiques, lesquelles sont largement utilisées en tant que conservateurs (JP-A-51091327) ou dans le traitement de pathologies comme l'acné où la composante microbienne joue un rôle clé dans l'étiologie (US 6123953). D'autres applications des alcanes diol 1,2 sont également décrites comme des propriétés protectrices vis-à-vis des odeurs corporelles grâce à leur effet anti-septique (US 5516510 ; WO 2003/000220) ou antimycosiques (WO 2003/069994). De même, des associations d'alcanes diol 1,2 avec d'autres composés sont décrites avec comme résultante un effet anti-microbien synergique. Ainsi, sont revendiquées dans ce dernier cadre des associations pour lutter contre les micro-organismes à l'origine des odeurs corporelles (US 2005/228032) ou impliqués dans la formation des lésions acnéiques (US 2007/265352, EP 1598064).

Les acides gras poly-insaturés (AGPI), quant à eux, se répartissent en deux catégories : les omégas 3 (ω3) et les omégas 6 (ω6). Outre leurs effets métaboliques, ils sont capables de modifier l'expression de gènes codant pour des protéines intracellulaires. Ces effets géniques des AGPI s'effectueraient via des récepteurs nucléaires appelés PPARS (peroxysome proliferator activated receptor). Les PPARS appartiennent à la famille des récepteurs nucléaires hormonaux de type stéroïdien. Ils forment des hétérodimères avec les récepteurs RXR (Retinoic X Receptor) de l'acide rétinoïque et modulent l'expression de gènes. Ainsi, les AGPI ω3 seraient des régulateurs négatifs de la réponse inflammatoire, en inhibant la voie d'activation NF-κB, par l'intermédiaire de l'induction de l'expression de IκBα, l'inhibiteur majeur de la voie NF-κB (Ren J and Chung SH. J Agric Food Chem. 2007 55: 5073-80). De plus, les AGPI ω3 ont une action inhibitrice sur la synthèse de l'acide arachidonique au profit de la synthèse des acides docosahexanoïques et eicosapentanoïques (Calder PC. Lipids : 2001; 36, 1007-24).

Au cours de l'acné, l'excès de sébum dans l'infundibulum pilaire représente un environnement propice pour la colonisation par *Propionibactérium acnes (P. acnes*). Une corrélation a ainsi pu être établie entre le degré de colonisation par *P. acnes* du canal pilo-sébacé et l'apparition du microcomédon. De plus, il a été montré que cette colonisation était plus importante chez les sujets acnéiques comparativement aux sujets sains (Brown S, Shalita A. Acne vulgaris. Lancet 1998 ; 351 : 1871-6).

*P. acnes* induit via les récepteurs de l'immunité innée, la production de cytokines pro-inflammatoires NF-κB dépendantes comme l'IL-8. Puis ces médiateurs vont influencer notamment la migration des polynucléaires neutrophiles vers le site inflammatoire où ils auront pour mission de tuer les bactéries. Cette réponse inflammatoire est un phénomène normal et nécessaire pour l'élimination du pathogène dans le tissu infecté. Cependant, une activation excessive et non régulée aboutit aux lésions inflammatoires acnéiques. Ainsi, il a été montré que le taux d'IL-8 est corrélé au nombre de neutrophiles mobilisés dans la lésion acnéique inflammatoire (Abd El All HS et al. Diagn. Pathol. 2007; 2: 4).

La colonisation bactérienne chez un patient acnéique est associée le plus souvent à l'apparition de lésions inflammatoires acnéiques : on retrouve par exemple un plus grand nombre de polynucléaires neutrophiles, mais aussi des taux plus élevés d'IL-8 au sein des canaux folliculaires fortement colonisés par rapport à des canaux faiblement colonisés chez des sujets non acnéiques. Les taux de ces marqueurs inflammatoires semblent corrélés à la charge bactérienne. Mais beaucoup de questions restent en suspens comme la cause de la colonisation, ou le mode de déroulement aboutissant à la formation des lésions. Quoi qu'il en soit, le rôle de la colonisation bactérienne comme facteur de progression de la maladie est avéré.

Le traitement actuel pour l'acné mineure à modérée ou inflammatoire correspond à une application topique d'actifs anti-bactériens agissant contre la colonisation notamment par *P. acnes* en association avec des anti-inflammatoires (Shalita A., J. Eur. Acad. Dermatol. Venereol. 2001 ; 15 : 43).

Les traitements sont initiés généralement après l'apparition d'un certain nombre de lésions inflammatoires acnéiques.

L'un des problèmes majeurs de la thérapie anti-acnéique reste de trouver un traitement adapté, c'est-à-dire proportionnelle à la gravité de l'acné, et débuté dès que possible, c'est à dire dès la primo-colonisation contre le développement de lésions acnéiques inflammatoires.

Les inventeurs ont ainsi découvert de manière surprenante que les esters d'alcane diol et d'acide gras polyinsaturé, et plus particulièrement d'omégas 3 ou 6, permettait d'avoir une action antibactérienne et anti-inflammatoire dès la primo colonisation par *P. acnes* dans le canal folliculaire, cette action étant par ailleurs proportionnelle à la colonisation.

En effet, il est apparu que les esters d'alcane diol et d'AGPI sont reconnus et clivés spécifiquement par la lipase bactérienne *(P. acnes*), permettant ainsi, par clivage de la liaison ester, la libération de deux actifs aux activités complémentaires, à savoir le diol antibactérien, capable de lutter contre la colonisation par *P. acnes,* et l'AGPI anti-inflammatoire, qui bloque le recrutement des neutrophiles et ainsi la cascade inflammatoire caractéristique de l'acné. La libération de ces deux actifs induit donc une réponse adaptée, c'est-à-dire proportionnelle à la colonisation par *P. acnes,* dès la primo colonisation et ainsi bloque l'évolution de cette pathologie, laquelle conduit à l'apparition de lésions acnéiques inflammatoires. Par ailleurs, la bactérie *P. acnes* étant présente chez des sujets qui ne présentent pas de lésions acnéiques, ces esters permettraient également de prévenir l'aggravation de la lésion acnéique en agissant dès la formation du comédon et en inhibant la cascade inflammatoire caractéristique de l'acné.

De tels esters ont déjà été décrits dans la littérature (WO 98/18751 ; Sugiura et al., J. Biol. Chem. 1999, 274(5), 2794-2801) mais pas pour leurs propriétés biologiques.

Ainsi la présente invention a pour objet un composé de formule générale (I) suivante : 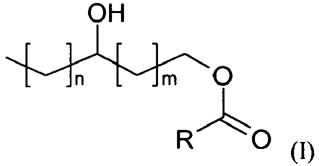 pour laquelle :
■ n représente un nombre entier compris entre 1 et 15, de préférence entre 1 et 10,
■ m représente 0, 1, 2 ou 3, et
■ R représente la chaîne hydrocarbonée d'un acide gras polyinsaturé choisi parmi les omégas 3 et les omégas 6.

Par « acide gras polyinsaturé », on entend, au sens de la présente invention, un acide carboxylique (R1CO₂H) linéaire comprenant de 10 à 28, de préférence de 16 à 24, et encore de préférence de 18 à 22, atomes de carbone (incluant l'atome de carbone de la fonction acide carboxylique) et comprenant au moins deux, de préférence 2 à 6, doubles liaisons C=C, ces doubles liaisons ayant de préférence une configuration *cis*.

Par « chaîne hydrocarbonée d'un acide gras polyinsaturé », on entend, au sens de la présente invention, la chaîne hydrocarbonée (R1) liée à la fonction acide de l'acide gras polyinsaturé (R1CO₂H). R1 représente donc une chaîne hydrocarbonée linéaire comprenant de 9 à 27, de préférence de 15 à 23, et encore de préférence de 17 à 21, atomes de carbone et comprenant au moins deux, de préférence 2 à 6, doubles liaisons C=C, ces doubles liaisons ayant de préférence une configuration *cis*. Ainsi, dans le cas de l'acide linoléique de formule suivante : 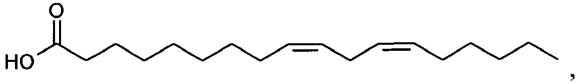 la chaîne hydrocarbonée considérée est la chaîne suivante :

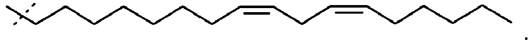

Par « oméga 3 », on entend, au sens de la présente invention, un acide gras polyinsaturé, tel que défini ci-dessus, pour lequel la première double liaison de la chaîne correspond à la troisième liaison carbone-carbone en comptant à partir de l'extrémité opposée à la fonction acide carboxylique, comme cela est illustré dans le cas de l'acide α-linolénique ci-dessous :

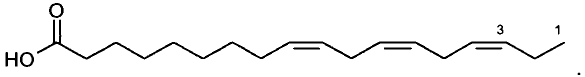

Les omégas 3 peuvent être en particulier l'acide α-linolénique, l'acide stéaridonique, l'acide eicosatriènoïque, l'acide eicosatetraènoïque, l'acide eicosapentaènoïque, l'acide docosapentaènoïque, l'acide docosahexaènoïque, l'acide tétracosapentaènoïque et l'acide tétracosahexaènoïque, et de préférence il s'agit de l'acide α-linolénique ou de l'acide stéaridonique, qui possèdent des propriétés anti-inflammatoires.

Par « oméga 6 », on entend, au sens de la présente invention, un acide gras polyinsaturé, tel que défini ci-dessus, pour lequel la première double liaison de la chaîne correspond à la sixième liaison carbone-carbone en comptant à partir de l'extrémité opposée à la fonction acide carboxylique, comme cela est illustré dans le cas de l'acide linoléique ci-dessous :

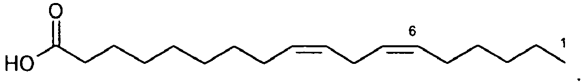

Les omégas 6 peuvent être en particulier l'acide linoléique, l'acide gamma-linolénique, l'acide eicosadiènoïque, l'acide dihomo-gamma-linolénique, l'acide arachidonique, l'acide docosatétraènoïque, l'acide docosapentaènoïque, l'acide adrénique et l'acide calendique, et de préférence il s'agit de l'acide linoléique, qui possède des propriétés séborégulatrices.

En particulier, n pourra représenter 1, 2, 3, 4 ou 5, et de préférence 5. Avantageusement, n ≥ 3 et de préférence n ≥ 5.

De manière avantageuse, m vaut 0 ou 1.

Avantageusement, n + m ≥ 3 et de préférence n + m ≥ 5.

Avantageusement, la chaîne hydrocarbonée provient d'un acide gras polyinsaturé choisi parmi l'acide α-linolénique, l'acide stéaridonique, l'acide eirosatriènoïque, l'acide eicosatetraènoïque, l'acide eicosapentaènoïque, l'acide docosapentaènoïque, l'acide docosahexaènoïque, l'acide tétracosapentaènoïque, l'acide tétracosahexaènoïque, l'acide linoléique, l'acide gamma-linolénique, l'acide eicosadiènoïque, l'acide dihomo-gamma-linolénique, l'acide arachidonique, l'acide docosatétraènoïque, l'acide docosapentaènoïque, l'acide adrénique et l'acide calendique. De préférence, l'acide gras polyinsaturé sera choisi parmi l'acide α-linolénique, l'acide stéaridonique et l'acide linoléique, et encore de préférence parmi l'acide α-linolénique et l'acide linoléique.

En particulier, les composés de l'invention peuvent être choisis parmi les molécules suivantes :

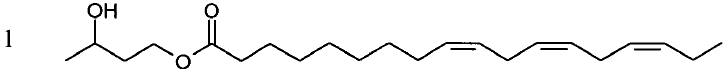

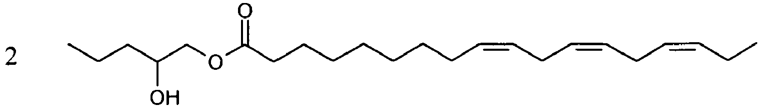

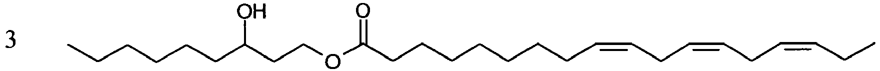

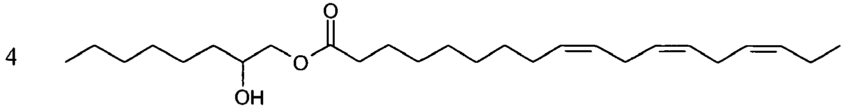

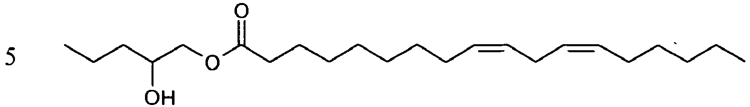

La présente invention a également pour objet un composé de formule (I) tel que défini ci-dessus pour son utilisation comme médicament, notamment destiné au traitement de l'acné ou de la dermite séborrhéique.

La présente invention concerne également l'utilisation d'un composé de formule (I) tel que défini ci-dessus, pour la fabrication d'un médicament, destiné notamment au traitement de l'acné ou de la dermite séborrhéique.

La présente invention concerne aussi une méthode de traitement de l'acné ou de la dermite séborrhéique comprenant l'administration d'une quantité efficace d'un composé de formule (I) tel que défini ci-dessus à une personne en ayant besoin.

La présente invention a également pour objet une composition pharmaceutique ou cosmétique comprenant au moins un composé de formule (I) tel que défini ci-dessus en association avec au moins un excipient pharmaceutiquement ou cosmétiquement acceptable, adapté notamment à une administration percutanée.

Dans la présente invention, on entend désigner par « pharmaceutiquement ou cosmétiquement acceptable » ce qui est utile dans la préparation d'une composition pharmaceutique ou cosmétique, qui est généralement sûr, non toxique et ni biologiquement ni autrement non souhaitable et qui est acceptable pour une utilisation thérapeutique ou cosmétique, notamment par application topique.

Les compositions pharmaceutiques ou cosmétiques selon l'invention pourront se présenter sous les formes qui sont habituellement connues pour une administration topique, c'est à dire notamment les lotions, les mousses, les gels, les dispersions, les émulsions, les shampooings, les sprays, les sérums, les masques, les laits corporels ou les crèmes, avec des excipients permettant notamment une pénétration cutanée afin d'améliorer les propriétés et l'accessibilité du principe actif. Avantageusement, il s'agira d'une crème.

Ces compositions contiennent généralement, outre le ou les composés selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants ou des eaux thermales, etc.

Ces compositions peuvent en outre contenir d'autres principes actifs conduisant à un effet complémentaire ou éventuellement synergique.

Avantageusement, les compositions selon la présente invention comprendront de 0,01 à 10% en poids, de préférence de 0, 1 % à 1% en poids, du ou des composés de formule (I) par rapport au poids total de la composition.

Ces compositions sont plus particulièrement destinées au traitement de l'acné ou de la dermite séborrhéique.

La présente invention a également pour objet une méthode de traitement cosmétique de l'acné ou de la dermite séborrhéique comprenant l'application sur la peau d'une composition cosmétique telle que définie ci-dessus.

La présente invention a également pour objet un procédé de préparation d'un composé de formule (I) tel que défini ci-dessus par couplage d'un acide gras polyinsaturé choisi parmi les omégas 3 et les omégas 6, dont la fonction acide carboxylique se trouve sous forme libre ou activée, et d'un diol de formule (II) suivante : 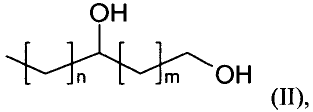 pour lequel n représente un nombre entier compris entre 1 et 15, et de préférence entre 1 et 10, et m représente 0, 1, 2 ou 3.

En particulier, n pourra représenter 1, 2, 3, 4 ou 5, et de préférence 5. Avantageusement, n ≥ 3 et de préférence n ≥ 5.

De manière avantageuse, m vaut 0 ou 1.

Avantageusement, n + m ≥ 3 et de préférence n + m ≥ 5.

Par « forme libre », on entend, au sens de la présente invention, que la fonction acide carboxylique de l'AGPI n'est pas protégée et représente donc un groupement CO₂H. L'AGPI est donc sous une forme R1CO₂H tel que définie précédemment.

Par « forme activée », on entend, au sens de la présente invention, une fonction acide carboxylique modifiée de manière à la rendre plus active vis-à-vis des nucléophiles. Ces formes activées sont bien connues de l'homme du métier et peuvent être en particulier un chlorure d'acide (COCl). L'AGPI activé sous forme d'un chlorure d'acide répond alors à la formule R1COCl.

Selon un premier mode de réalisation particulier de l'invention, l'acide gras polyinsaturé est utilisé sous sa forme acide libre. Dans ce cas, la réaction de couplage sera réalisée en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-H-benzotriazole-1-yl)-1,1,3,3-tetramethyluronium hexafluorophosphate (HBTU), le 2-(1H-benzotriazole-1-yl)-1,1,3,3-tetramethyluronium tetrafluoroborate (TBTU) ou le O-(7-azobenzotriazol-1-yl)-1,1,3,3-tétraméthyluronium hexafluorophosphate (HATU), éventuellement associé à un auxiliaire de couplage, tel que le N-hydroxy succinimide (NHS), le N-hydroxy benzotriazole (HOBt), le 3,4-dihydro-3-hydroxy-4-oxo-1,2,3-benzotriazole (HOOBt), le 1-hydroxy-7-azabenzotriazole (HOAt), la diméthylaminopyridine (DMAP) ou le N-hydroxysylfosuccinimide (sulfo NHS). Avantageusement, le couplage sera réalisé en présence d'un carbodiimide (notamment DIC, DCC ou EDC) et de diméthylaminopyridine. De préférence, le couplage sera réalisé en présence de chlorhydrate de 1-(3-diméthylaminopropyl)-3-éthylcarbodiimide ou de diisopropylcarbodiimide et de diméthylaminopyridine.

Selon un second mode de réalisation particulier de l'invention, l'acide gras polyinsaturé est utilisé sous sa forme activée, et plus particulièrement sous forme d'un chlorure d'acide. Dans ce cas, la réaction de couplage sera avantageusement réalisée en présence de pyridine et de diméthylaminopyridine.

La présente invention sera mieux comprise à la lumière des exemples non limitatifs qui suivent.

### DESCRIPTION DES FIGURES :

Figure 1 : Mise en évidence de la lipase *P. acnes* (33 kDa) dans les lésions acnéiques inflammatoires (M correspond à des marqueurs de taille ; A correspond aux lésions acnéiques inflammatoires ; B correspond aux comédons hors inflammation).
Figure 2 : Etude de l'hydrolyse des esters de l'invention par la lipase recombinante du *P. acnes* après 2h d'incubation.
   La colonne gris clair représente les résultats obtenus avec l'alpha-linolénate de 3-hydroxybutyle, la colonne gris foncé représente les résultats obtenus avec l'alpha-linolénate de 2-hydroxypentyle, la colonne noire représente les résultats obtenus avec l'alpha-linolénate de 2-hydroxyoctyle, la colonne blanche représente les résultats obtenus avec l'alpha-linolénate de 3-hydroxynonyle et la colonne hachurée représente les résultats obtenus avec le trilinolénate de glycéryle.

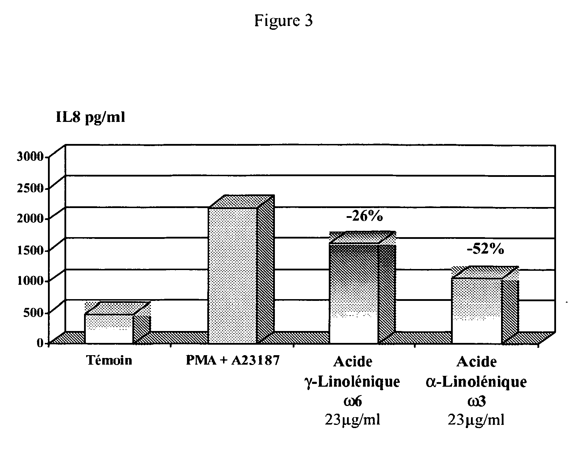

Figure 3 : Activité des AGPI sur la libération d'interleukine 8 par les kératinocytes humains HaCaT stimulés par PMA/A23187 (agents pro-inflammatoires).

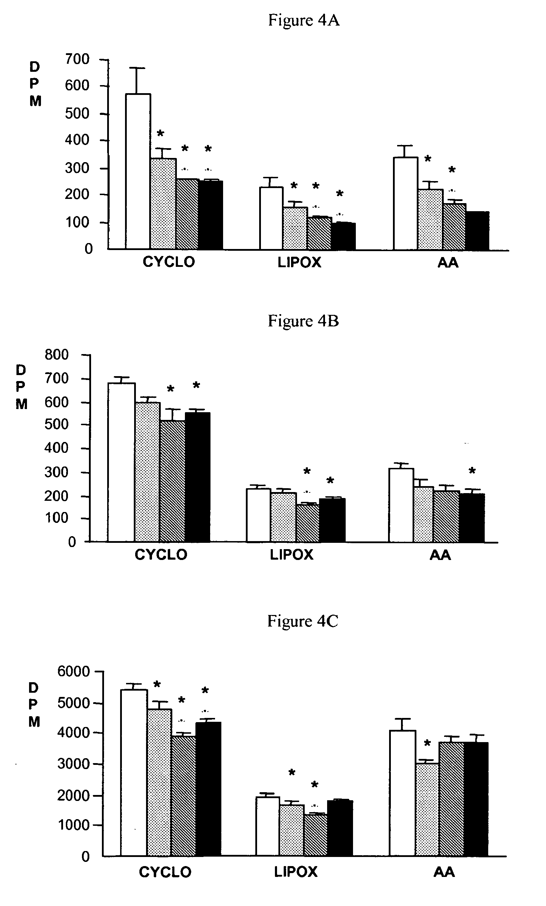

Figures 4A, 4B et 4C : Effets des AGPI sur la production d'éicosanoïdes (métabolites cyclooxygénés (CYCLO) ; métabolites lipoxygénés (LIPOX) ; acide arachidonique (AA)) au cours d'une réponse inflammatoire induite par PMA/A23187.

Les colonnes blanches représentent le témoin (c'est-à-dire un milieu réactionnel sans AGPI) ; les colonnes gris clair correspondent à l'activité de l'AGPI à 2,3 µg/mL ; les colonnes gris foncé correspondent à l'activité de l'AGPI à 11,5 µg/mL et les colonnes noires correspondent à l'activité de l'AGPI à 23 µg/mL.

La Figure 4A correspond à l'acide α-linolénique, la Figure 4B correspond à l'acide stéaridonique et la Figure 4C correspond à l'acide linoléique.

### ABREVIATIONS UTILISEES :

| | |
|---|---|
| APCI | Ionisation Chimique à Pression Atmosphérique |
| CMI | Concentration Minimale Inhibitrice |
| DPM | Désintégration par Minute |
| ESI | Ionisation par Electrospray |
| HPLC | Chromatographie Liquide Haute Performance |
| RMN | Résonance Magnétique Nucléaire |
| Rf | Rapport au front |
| SM | Spectre de Masse |
| UFC | Unité Formant Colonie |

### EXEMPLE 1 : Synthèse des composés de l'invention

### 1.1. Procédé général A : à partir de l'acide gras insaturé

L'acide gras insaturé est mis en solution dans 20ml de CH₂Cl₂ anhydre sous circulation de N₂. Un carbodiimide (agent de couplage) (1,1 éq.) et la diméthylaminopyridine (0,5 éq.) sont ensuite ajoutés directement. Après agitation du milieu à température ambiante pendant 5-10 minutes, le diol (5 éq.) est ajouté. Le milieu est agité vigoureusement pendant 18 heures, sous N₂, à l'abri de la lumière. La phase organique est alors extraite avec CH₂Cl₂ puis lavée avec une solution saturée de NaCl, séchée sur MgSO₄, filtrée et concentrée.
Le produit brut est une huile jaune qui est purifiée par chromatographie ouverte, gel de silice (Ø : 22x3,5cm), conditionnement CHCl₃.

Les trois produits suivants ont été préparés selon le procédé A avec le diisopropylcarbodiimide comme agent de couplage.

### (9Z,12Z,15Z)-Octadéca-9,12,15-triénoate de 3-hydroxybutyle (C₂₂H₃₈O₃) (α-linolénate de 3-hydroxybutyle)

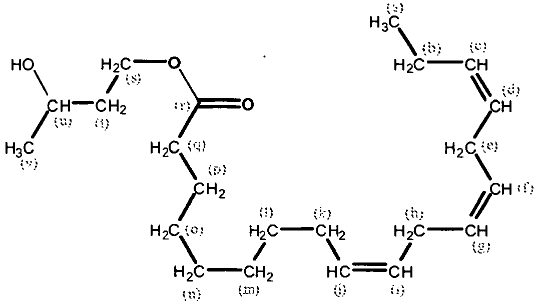

Conditions de purification : gradient d'élution : CHCl₃ / AcOEt : 95/5 puis CHCl₃ /AcOEt : 9/1
Huile translucide (rendement : 69%)
**Rf** (CHCl₃ / AcOEt : 9/1) = 0,5
**RMN (¹H, CDCl₃)** δ (ppm) : 0.98 (t, 3H, CH₃₍ₐ₎) ; 1.22 (d, 3H, CH₃₍ᵥ₎); 1.31 (m, 8H, CH_{2 (o, n, m, l)}) ;1.6 (m, 2H, CH_{2 (p)}) ; 1.8 (m, 2H, CH_{2 (t)}) ; 2.1 (m, 4H, CH_{2 (k,b)}) ; 2.3 (t, 2H, CH_{2 (q)}) ; 2.8 (m, 4H, CH_{2 (h, e)}) ; 3.85 (m, 1H, CH ₍ᵤ₎) ; 4.1-4.3 (m, 2H, CH_{2 (s)}) ; 5.4 (m, 6H, CH _{(c,d,f,}g_{,i,j)}).
**RMN (¹³C, CDCl₃)** δ (ppm) : 14.24 (CH_{3 (a)}) ; 20.46 (CH_{2 (b)}) ; 23.42 (CH_{3 (v)}) ; 25.01 (CH_{2 (p)}) ; 25.5 (CH_{2 (e)}) ; 25.59 (CH_{2 (h)}) ; 27.16 ( CH_{2 (k)}) ; 29.06 (CH_{2 (o, n)} , 29.07 (CH_{2 (m)}) , 29.53 (CH_{2 (1)}) , 34.3 (CH_{2 (q)}) , 38.1 (CH_{2 (t)}) , 61.51 (CH_{2 (s)}) , 64.89 (CH ₍ᵤ₎) , 127.08 (CH _{(c)}) , 127.6 (CH ₍ⱼ₎) , 128.22 (CH _{(f)}) , 128.26 (CH _{(g)}) , 130.22 (CH ₍ᵢ₎) , 131.93 (CH _{(d)}) , 174.2 (C=O ₍ᵣ₎).
**SM : ESI+** [M+H]⁺ = 351,2 (100%) ; [M+Na]⁺ = 373,3 (48%)
**APCI+** [M+H]⁺ = 351,2 (M calculé = 350,2)

### (9Z,12Z,15Z)-Octadéca-9,12,15-triénoate de 2-hydroxypentyle (C₂₃H₄₀O₃) (α-linolénate de 2-hydroxypentyle)

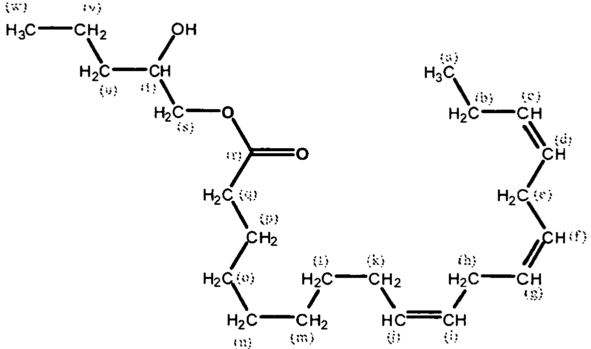

Conditions de purification : CHCl₃ / AcOEt : 98/2
Huile translucide (rendement : 45%)
Rf (CHCl₃ / AcOEt : 97/3) = 0,58
**RMN (¹H, CDCl₃)** δ (ppm) : 1 (m, 6H, CH_{3(w, a)}) ; 1.25 (m, 10H, CH_{2 (o, n, m, l, v,)}) ; 1.5 (m, 4H, CH_{2 (p,u)}) ; 1.65 (m, 4H, CH_{2 (b,k)}) ; 2.1 (m, 2H, CH_{2 (e)}) ; 2.4 (t, 2H, CH_{2 (q)}) ; 2.8 (m, 2H, CH_{2 (h)}) ; 3.9 (m, 1H, CH ₍ₜ₎) ; 4.1-4.3 (m, 2H, CH_{2 (s)}) ; 5.4 (m, 6H, CH _{(c,d,f,g,i,j)}).
**RMN (¹³C, CDCl₃)** δ (ppm) : 14.2 (CH_{3 (a, w)}) ; 18.6 (CH_{2 (v, b)}) ; 25.5 (CH_{2 (p)}) ; 25.5 (CH_{2 (p)}) ; 27.16 (CH_{2 (e)}) ; 28.9 ( CH_{2 (h)}) ; 29.1 (CH_{2 (k)}) , 29.4 (CH_{2 (o,n)}) , 29.5 (CH_{2 (m)}) , 34.17 (CH_{2 (q)}) , 35.41 (CH_{2 (u)}) , 68.53 (CH ₍ₜ₎) , 69.76 (CH_{2 (s)}) , 128.22 (CH _{(c)}) , 129.58 (CH ₍ⱼ₎) , 130.22 (CH _{(f)} CH _{(g)}) , 130.81 (CH ₍ᵢ₎) , 131.93 (CH _{(d)}) , 174.05 (C=O ₍ᵣ₎).
**SM : APCI+** [M+H]⁺ = 365,1 (M calculé = 364,3)

### (9Z, 12Z, 15Z)-Octadéca-9,12,15-triénoate de 3-hydroxynonyle (C₂₇H₄₈O₃) (α-linolénate de 3-hydroxynonyle)

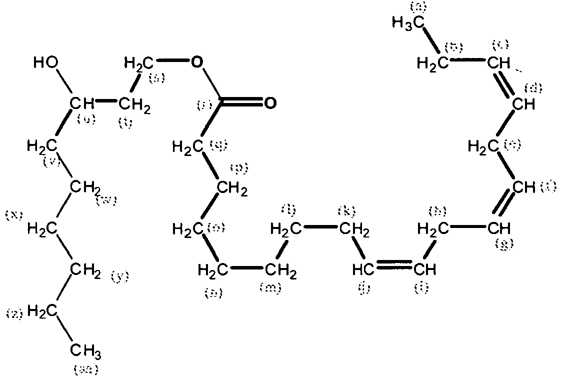

Conditions de purification : CHCl₃ / AcOEt : 96/4
Huile translucide (rendement 73%)
**Rf** (CHCl₃ / AcOEt : 97/3) = 0,64
**RMN (¹H, CDCl₃)** δ (ppm) : 0.95 (m, 3H, CH₃₍ₐₐ₎) 1 (m, 3H, CH₃₍ₐ₎) ; 1.4 (m, 16H, CH_{2 (o, n, m, l, w, x, y, z)}) ;1.5 (m, 2H, CH_{2 (v)}) ; 1.6-1.8 (m, 4H, CH_{2 (p, t)}) ; 2 (m, 4H, CH_{2 (k,b)}) ; 2.3 (t, 2H, CH_{2 (q)}) ; 2.8 (m, 4H, CH_{2 (h,e)}) ; 3.7 (m, 1H, CH ₍ᵤ₎) ; 4.1 - 4.3 (m, 2H, CH_{2 (s)}) ; 5.4 (m, 6H, CH _{(c,d,f,g,i,j)}).
**RMN (¹³C, CDCl₃)** δ (ppm) : 14 (CH_{3 (a ; aa)}) ; 20.4 (CH_{2 (b)}) ; 22.5 (CH_{2 (z)}) ; 25.09 (CH_{2 (p)}) ; 25.26 (CH_{2 (w)}) ; 25.58 (CH_{2 (e,h)}) ; 27.17 ( CH_{2 (k)}) ; 29.06-29,3 (CH_{2 (x, o, n,,m,l)}) ,31.7 (CH_{2 (y)}) ,34.5 (CH_{2 (q)}) , 37.45 (CH_{2 (t)}) , 37.58 (CH_{2 (v}) , 61.59 (CH_{2 (s)}) , 68.72 (CH ₍ᵤ₎) ,127.08 (CH _{(c)}) , 127.71 (CH ₍ⱼ₎) , 128.21 (CH _{(f)}) , 128.26 (CH _{(g)}) , 130.22 (CH ₍ᵢ₎) , 131.94(CH _{(d)}) , 174.9 (C=O ₍ᵣ₎.
**SM : ESI+** [M+H]⁺ = 421,1 (100%) ; [M+Na]⁺ = 443,1 (92%)

Le produit suivant a été préparé selon le procédé A avec le chlorhydrate de 1-(3-diméthylaminopropyl)-3-éthylcarbodiimide comme agent de couplage.

### 9Z,12Z,15Z)-Octadéca-9,12,15-triénoate de 2-hydroxyoctyle (C₂₆H₄₆O₃) (α-linolénate de 2-hydroxyoctyle)

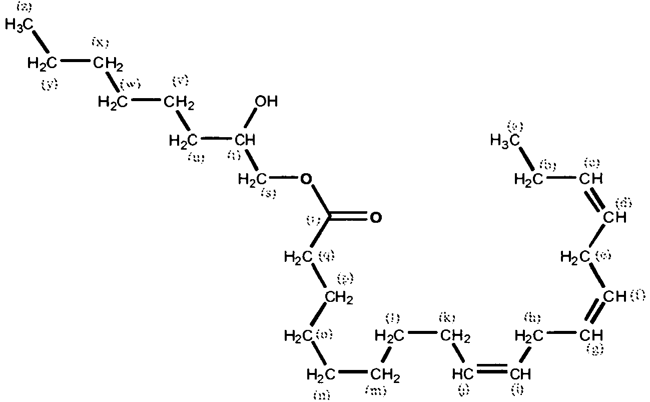

Conditions de purification : CHCl₃ / AcOEt : 98/2
Huile translucide (rendement 37%)
**Rf** (CHCl₃ / AcOEt : 98/2) = 0,72
**RMN (¹H, CDCl₃)** δ (ppm) : 0.88 (m, 3H, CH_{3(z)}) , 0.97 (t, 3H, CH₃₍ₐ₎) ; 1.31 (m, 16H, CH_{2 (o, n, m, l, v, w, x, y)}) ; 1.47 (m, 2H, CH_{2 (u)}) ; 1.65 (m, 2H, CH_{2 (p)}) ; 2.02 (m, 4H, CH_{2 (b,k)}) ; 2.4 (t, 2H, CH_{2 (q)}) ; 2.8 (m, 4H, CH_{2 (e,h)}) ; 3.9 (m,1H, CH ₍ₜ₎) ; 4.1-4.3 (m, 2H, CH_{2 (s)}) ; 5.4 (m, 6H, CH _{(c,d,f,g,i,j)}).
**RMN (¹³C, CDCl₃)** δ (ppm) : 14.2 (CH_{3 (a, w)}) ; 18.6 (CH_{2 (v, b)}) ; 22.7 (CH_{2 y)}) , 25.5 (CH_{2 (p)}) ; 25.5 (CH_{2 (p)}) ; 27.16 (CH_{2 (e)}) ; 28.9 ( CH_{2 (h)}) ; 29.1 (CH_{2 (k)}) , 29.4 (CH_{2 (o,n)}) , 29.5 (CH_{2 (m)}) , 29.9 (CH_{2 (w)}), 31.7 (CH_{2 (x)}), 34.17 (CH_{2 (q)}) , 35.41 (CH_{2 (u)}), 68.53 (CH ₍ₜ₎) , 69.76 (CH_{2 (s)}) , 128.22 (CH _{(c)}) , 129.58 (CH ₍ⱼ₎) , 130.22 (CH _{(f)} CH _{(g)}) , 130.81 (CH ₍ᵢ₎) , 131.93 (CH _{(d)}), 174.05 (C=O ₍ᵣ₎).
**SM : APCI+** [M+H]⁺ = 407,3 (M calculé = 406,34)

### 1.2. Procédé général B : à partir d'un chlorure d'acide gras polyinsaturé

Le chlorure de linoléyle (6,7.10⁻³ mol) et la diméthylaminopyridine (DMAP) (0,1 éq.) sont ajoutés, sous circulation d'azote, à une solution de diol (5 éq.) dans la pyridine (20 ml). Après agitation vigoureuse pendant 18 heures sous N₂, à température ambiante et à l'abri de la lumière, le milieu réactionnel est ramené à sec.
La phase organique est ensuite extraite avec AcOEt puis lavée avec H₂O et NH₄⁺Cl⁻, séchée sur MgSO₄, filtrée et concentrée.
Le produit brut est une huile marron qui est purifiée par chromatographie ouverte, gel de silice (Ø : 22x3.5cm), conditionnement CHl₃.
Le produit suivant a été préparé selon le procédé B avec le pentylène glycol comme diol.

### (9Z, 12Z)-Octadéca-9,12-diénoate de 2-hydroxypentyle (C₂₃H₄₂O₃) (linoléate de 2-hydroxypentyle)

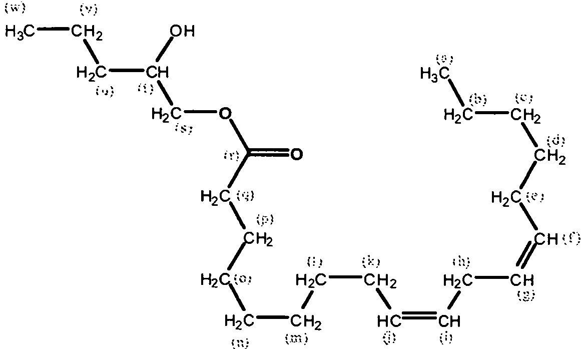

Conditions de purification : CHCl₃ / AcOEt : 98/2
Huile translucide
**Rf** (CHCl₃ / AcOEt : 98/2) = 0,54
**RMN (¹H, CDCl₃)** δ (ppm) : 0.9 (m, 6H, CH_{3(a ; w)}) 1.3 (m, 16H, CH_{2 (o, n,} m, _{l, b, c, d, v)}) ; 1.45 (m, 2H, CH_{2 (u)}) ; 1.65 (m, 2H, CH_{2 (p)}) ; 2 (m, 4H, CH_{2 (k,e)}) ; 2.3 (t, 2H, CH_{2(q)}) ; 2.8 (m, 2H, CH_{2 (h)}) ; 3.8 (m, 1H, CH ₍ₜ₎) ; 4.1 - 4.3 (m, 2H, CH_{2 (s)}) ; 5.4 (m, 4H, CH _{(f,g,i,j)}).
**RMN (¹³C, CDCl₃)** δ (ppm) : 14.2 (CH_{3 (a, w)}) ; 18.6 (CH_{2 (v)}) ; 22.6 (CH_{2 (b)}), 25 (CH_{2 (p)}), 25.6 (CH_{2 (h)}), 27.2 (CH_{2 (e,h)}), 29 (CH_{2 (o)}) ; 29.1 (CH_{2 (n)}) ; 29.3 (CH_{2 (d)}) ; 29.4 ( CH_{2 (m)}) ; 29.6 (CH_{2 (l)}) , 31.4 (CH_{2 (c)}) , 34.1 (CH_{2 (q)}) , 36.2 (CH_{2 (u)}) , 69 (CH_{2 (s)}) , 70 (CH ₍ₜ₎) , 127.78 (CH _{(g)}) , 127.91 (CH ₍ᵢ₎) , 129.94 (CH _{(f)}) , 130.22 (CH ₍ⱼ₎) , 174.05 (C=O ₍ᵣ₎).
**SM : ESI+** [M+H]⁺ = 367,3 (100%) ; [M+Na]⁺ = 389,3 (80%)

### EXEMPLE 2 : Composition selon l'invention

Une formulation selon l'invention, sous forme de crème, a la composition suivante (les quantités sont données en pourcentages massiques par rapport au poids total de la composition) :

| **Quantité** | **Désignation** | **Fonction** |
|---|---|---|
| 3 | Glycérine | Humectant |
| 0,1 | EDTA^{*} disodique | Agent complexant |
| 0,35 | Phénoxyéthanol | Conservateur |
| 1 | Polyacrylate-13 ; Polyisobutène ; Polysorbate 20 ; eau (mélange commercialisé sous le nom Sepiplus^{®} 400 par SEPPIC) | Agent gélifiant et stabilisant |
| 4 | Stéarate de glycéryle ; PEG-100 stéarate (mélange commercialisé sous le nom Simulsol^{®} 165 par SEPPIC) | Emulsionnant |
| 1 | Alcool cétylique | Facteur de consistance |
| 5 | Cyclopentasiloxane | Emollient |
| 3 | Tri-2-éthylhexanoate de glycéryle | Emollient |
| 2 | Carbonate de dicaprylyle | Emollient |
| 1 | Ester de diol et d'AGPI selon l'invention | Agent antiacnéique |
| 0,27 | Chlorphénésine | Conservateur |
| 2 | Polymméthylméthacrylate | Poudre matifiante |
| 0,1 | Parfum | Parfum |

| | | |
|---|---|---|
| ^{*} EDTA = acide éthylène-diamine-tétraacétique | | |

### EXEMPLE 3 : Résultats des tests biologiques

### 3.1. Etude de l'action de la lipase P. acnes sur les composés de l'invention

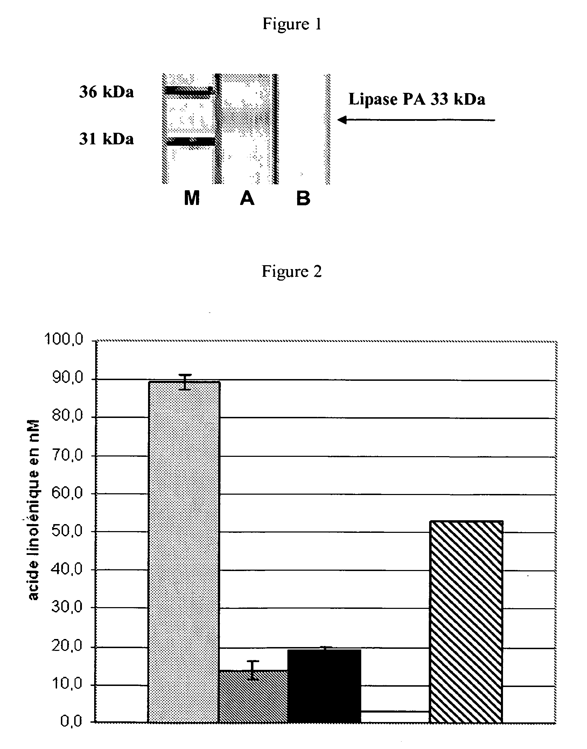

Dans un premier temps il a été démontré, par western blot, que la lipase du *P. acnes* est fortement induite dans les lésions inflammatoires de sujets acnéiques comparativement à des prélèvements effectués chez des sujets sains (voir Figure 1).
Dans un second temps, nous avons procédé à l'étude de l'action de la lipase recombinante du *P. acnes* sur les composés de l'invention, comme décrit ci-dessous.
La réaction enzymatique a été réalisée en incubant le substrat à concentration souhaitée (ici, 500 µM) dans le tampon TRIS (laissé à température ambiante 1h avant le début des essais) puis initiée par addition de l'enzyme et incubation à 37°C. Les essais sont réalisés dans des vials en verre ambré de 2 ml. Un contrôle est réalisé en ne mettant pas d'enzyme pour suivre l'hydrolyse spontanée éventuelle du substrat dans le tampon. Une prise d'essai de 10µl est réalisée à chaque temps souhaité au cours de la cinétique puis congelé pour stopper la réaction. L'acide linolénique libéré est alors dérivatisé avec l'Antry-Idiazométhane (ADAM) qui forme un complexe fluorescent avec l'acide gras. Le produit formé est alors séparé par HPLC puis quantifié à l'aide d'une gamme étalon d'acide linolénique commercial dérivatisé dans les mêmes conditions.
Comme le montre la Figure 2, nous avons pu montrer que les composés de l'invention donnent lieu à une réaction d'hydrolyse en présence de *P. acnes* puisque l'on observe la formation d'acide α-linolénique.
Ainsi, l'hydrolyse des composés de l'invention par *P. acnes* permet de libérer le diol d'une part et l'AGPI d'autre part. L'expression de la lipase est donc une condition nécessaire pour observer le clivage des composés de l'invention et donc pour obtenir un effet thérapeutique. Par ailleurs, la lipase *P. acnes* étant essentiellement présente chez les sujets acnéiques comme le montre la Figure 1, les composés de l'invention devrait permettre une réponse adaptée pour chaque personne.

### 3.2. Etude de l'activité antibactérienne des diols obtenus après hydrolyse des composés de l'invention

Ces diols répondent à la formule générale (II) suivante : 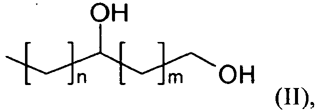 avec n représentant un nombre entier compris entre 0 et 15 et m représentant 0, 1, 2 ou 3.

Des tests ont été réalisés avec 8 souches microbiennes en utilisant la méthode de dilution décrite ci-dessous.

Les CMI sont déterminées par microméthode en milieu liquide. Les dilutions successives de raison 2 des produits tests dans le bouillon de culture (Trypcase Soja) sont réalisées en microplaques à 96 puits dans un volume final de 0,1 ml. Les puits sont ensemencés avec 0,01 ml des suspensions bactériennes titrées à environ 10⁷ UFC/ml. Les microplaques sont incubées en conditions optimales de croissance et la CMI est lue visuellement.

Le tableau 1 suivant présente les résultats obtenus avec différents diols. Les colonnes « STASE » représentent l'activité bactériostatique et les colonnes « CIDIE » représentent l'activité bactéricide des diols (1% correspond à une concentration de 10 mg/ml).

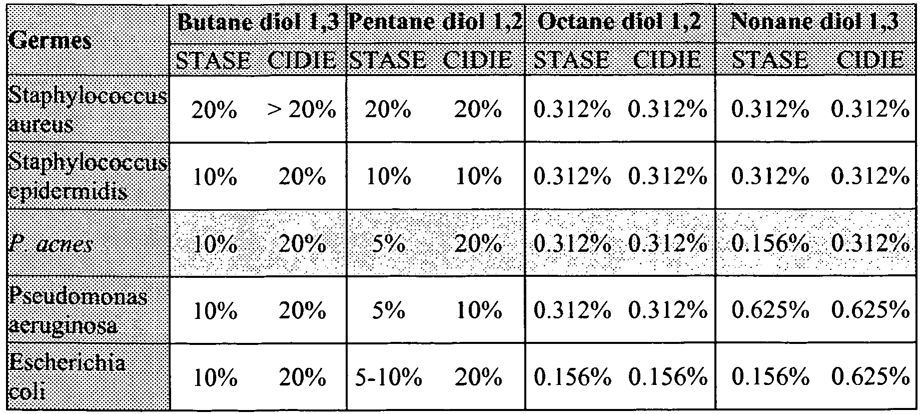

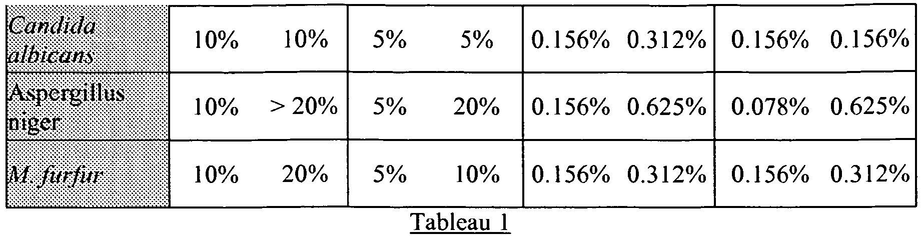

L'effet antibactérien observé est en conformité avec la littérature à savoir que l'activité est en relation avec la longueur de chaîne du diol (Frankenfeld JW, Mohan RR, Squibb RL. J Agric Food Chem. 1975 ; 23 : 418-425). Ainsi, le nonane-diol 1,3 et l'octane-diol 1,2 sont bactériostatiques et bactéricides (gram+, gram-, fongiques) dans une gamme de concentration allant de 6,25 à 0,78 mg.ml⁻¹. Il est à noter que le dérivé nonane apparaît comme celui présentant l'activité la plus marquée pour la souche *P. acnes* avec notamment un effet bactériostatique et bactéricide trouvé respectivement à 1,56 mg.ml⁻¹ et 3,12 mg.ml⁻¹.

### 3.3. Etude cinétique de l'activité antibactérienne de l'octane diol 1,2

L'étude cinétique de l'activité antibactérienne de l'octane diol 1, 2 a été réalisée en évaluant la létalité des bactéries au cours du temps par dénombrement des UFC (Unités Formant Colonie) résiduelles après contact de la solution test avec la suspension bactérienne.
Les souches bactériennes utilisées sont : *Staphylococcus aureus* ATCC6538 et *Propionibacterium acnes* ATCC6919. Le milieu de culture utilisé pour *Staphylococcus aureus* ATCC6538 est la Gélose Trypcase Soja tandis que le milieu de culture utilisé pour *Propionibacterium acnes* ATCC6919 est la gélose de Schaedler. Des solutions mères contenant 1% et 0,5% d'octane diol 1,2 sont préparées extemporanément dans 1 % de Polysorbate 20 (qsp eau PPI) puis ajustées à pH 7 avec une solution de NaOH 0,1N.
Un volume de 5ml de chacune des solutions mères est ensemencé avec 50µl de suspension bactérienne titrant environ 1.10⁸UFC/ml.
Après chaque temps de contact (temps de contacts testés : 5mn, 15mn, 30mn et 60mn), un dénombrement des bactéries résiduelles est effectué par prise d'un aliquot de 1ml neutralisé par 9ml de Bouillon Trypcase Soja et 10% de Polysorbate 80.
Le dénombrement est réalisé par inclusion de 1ml du mélange neutralisé et de 4 dilutions sériées au 1/10 dans les géloses de culture correspondantes.
Les géloses sont incubées à 36°C +/- 1°C en atmosphère aérobie pour *S*. *aureus* et anaérobie pour *P. acnes* (Generbag Anaer, Biomérieux) pendant 72 heures puis les UFC sont dénombrées.
Le tableau 2 suivant présente les résultats obtenus.

**Tableau 2**

| | | *Staphylococcus aureus* Log R | | | | *Propionibacterium acnes* Log R | | | |
|---|---|---|---|---|---|---|---|---|---|
| Concentration | pH | 5mn | 15mn | 30mn | 60mn | 5mn | 15mn | 30mn | 60mn |
| 1% | 7 | >5,3 | >5,3 | >5,3 | >5,3 | >5 | >5 | >5 | >5 |
| 0,5% | 7 | 0,4 | 0,1 | 0,2 | 0,5 | 0,2 | 0,7 | 1,8 | 3 |
| Témoin négatif: Excipient Polysorbate 20 à 1% | 6,5 | 0 | 0 | 0 | 0,1 | 0 | 0 | 0,2 | 0,3 |

- Avec R signifie « Réduction ». Il s'agit du rapport entre le nombre de bactéries inoculées et le nombre de bactéries résiduelles après contact de la solution test avec la suspension bactérienne.
L'action bactéricide de l'octane diol a été examinée sur deux espèces bactériennes *(P. acnes, S. aureus*) aux deux concentrations suivantes 0,5% et 1%. A pH physiologique et à la concentration la plus basse (0,5 %), une décroissance supérieure à 99,9 % de la population notamment du *P. acnes* est observée après un temps de contact de 1 heure.

### 3.4. Etude de l'activité anti-inflammatoire des AGPI obtenus après hydrolyse des composés de l'invention

Le potentiel anti-inflammatoire des AGPI a été testé sur des kératinocytes humains (HACAT) stimulés par du PMA/A23187. Les résultats obtenus sont présentés sur les Figures 3, 4A, 4B et 4C.
Les changements induits par ces AGPI sont évalués sur la production des eicosanoïdes et d'une cytokine inflammatoire, l'Interleukine 8, au cours d'un processus inflammatoire déclenché par PMA/A23187.

### Etude de la sécrétion d'IL8 (Figure 3) :

Les cellules HaCaT sont incubées sur plaques 96 puits pendant 24h en présence des AGPI, rincées puis stimulées par PMA/A23187 ; les surnageants sont récupérés au bout de 6 heures de stimulation et l'IL8 est quantifiée en kit ELISA OptEIA™ commercialisé par BD Pharmingen.

### Etude du métabolisme de l'acide arachidonique (AA) (Figures 4A, 4B, 4C) :

Les kératinocytes HaCaT ont été incubés en plaque 24 puits en présence de 1 µCi [³H]AA durant 18 heures sous une atmosphère de 5% CO₂. Pour tester la capacité des AGPI à moduler la réponse inflammatoire des kératinocytes, les HaCaT [³H] marqués sont prétraités avec les AGPI pendant 24 heures, rincés puis stimulés par 500 nM de PMA et 1 µM d'A23187 durant 5 heures. Le [³H]AA et les métabolites relâchés par les HaCat dans le milieu de culture sont extraits par chromatographie sur colonnes. L'éluat est évaporé sous N₂. Le résidu sec est repris par du méthanol et déposé sur des plaques de silices en couches minces préalablement activées à 100°C pendant 1h. Le solvant de migration est la phase organique du mélange acétate d'éthyle / eau / isooctane / acide acétique (110:100:50:20, v/v). L'AA [³H] et les métabolites (6-keto-ProstaGlandine F_{1α} , 6k-PGF_{1α} ; ProstaGlandine (PG) FαE2D₂,Thromboxane (TX) B₂, Leukotrienes (LT) B₄, C₄, D₄) sont identifiés par un scanner BERTHOLD TLC.
Ainsi, la Figure 3 montre l'activité de certains AGPI sur la libération d'interleukine 8 (IL-8) par les kératinocytes humains HaCaT stimulés par PMA/A23187. Un effet inhibiteur sur la sécrétion d'IL-8 des kératinocytes stimulés est clairement démontré pour l'acide γ-linolénique et particulièrement pour l'acide α-linolénique.

De même, la pré-incubation des kératinocytes avec un AGPI, tel que l'acide α-linolénique, l'acide stéaridonique ou l'acide linoléique, inhibe significativement la libération d'acide arachidonique et également celles des métabolites cyclooxygénés et lipoxygénés de la cascade inflammatoire (Figures 4A, 4B et 4C).

Ainsi, l'ensemble des résultats obtenus confirment l'activité anti-inflammatoire des acides gras polyinsaturés ω-3 ou ω-6 et en particulier de l'acide α-linolénique.

Les composés selon l'invention sont donc utiles pour le traitement de l'acné. Le schéma étiologique de la dermite séborrhéique présentant des similitudes avec celui de l'acné, les composés de l'invention sont également potentiellement utiles pour le traitement de la dermite séborrhéique. En effet, le microorganisme à l'origine de la réponse inflammatoire dans le cadre de cette pathologie, le *Malassezia,* sécrète des lipases (DeAngelis YM et al. J Invest Dermatol. 2007 ;127: 2138-46) au niveau du cuir chevelu qui peuvent également être mises à profit pour libérer les deux actifs, l'alcane diol aux propriétés anti-fongiques et l'AGPI tel que l'acide α-linolénique aux propriétés anti-inflammatoires. En outre, certains acides gras comme l'acide linoléique sont des activateurs des récepteurs nucléaires PPARα, lesquels exercent un effet séborégulateur marqué (Downie MM et al. Br J Dermatol. 2004 ; 151:766-75). Ainsi, les conjugués diol-acide linoléique peuvent être utilisés en particulier pour limiter la séborrhée dans diverses indications comme l'acné et la dermite séborrhéique.

## Revendications

1. Composé de formule générale (I) suivante : 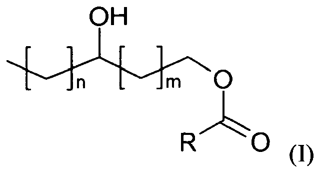 pour laquelle :
■ n représente un nombre entier compris entre 1 et 15,
■ m représente 0, 1, 2 ou 3, et
■ R représente la chaîne hydrocarbonée d'un acide gras polyinsaturé choisi parmi les omégas 3 et les omégas 6.

2. Composé selon la revendication 1, **caractérisé en ce que** n est compris entre 1 et 10.

3. Composé selon l'une quelconque des revendications 1 et 2, **caractérisé en ce que** n représente 1, 2, 3, 4 ou 5.

4. Composé selon l'une quelconque des revendications 1 à 3, **caractérisé en ce que** m représente 0 ou 1.

5. Composé selon l'une quelconque des revendications 1 à 4, **caractérisé en ce que** l'acide gras polyinsaturé est choisi parmi l'acide α-linolénique, l'acide stéaridonique, l'acide éicosatriènoïque, l'acide éicosatetraènoïque, l'acide éicosapentaènoïque, l'acide docosapentaènoïque, l'acide docosahexaènoïque, l'acide tétracosapentaènoïque, l'acide tétracosahexaènoïque, l'acide linoléique, l'acide gamma-linolénique, l'acide éicosadiènoïque, l'acide dihomo-gamma-linolénique, l'acide arachidonique,l'acide docosatétraènoïque, l'acide docosapentaènoïque, l'acide adrénique et l'acide calendique, et de préférence est l'acide α-linolénique, l'acide stéaridonique ou l'acide linoléique.

6. Composé selon l'une quelconque des revendications 1 à 5, **caractérisé en ce qu'**il est choisi parmi les molécules suivantes :

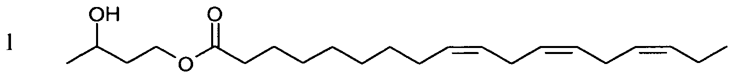

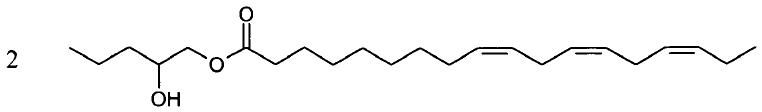

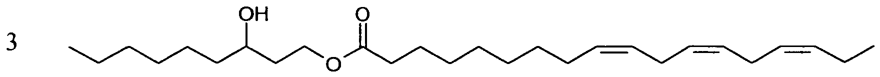

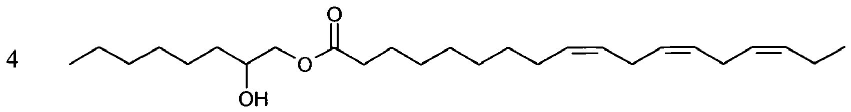

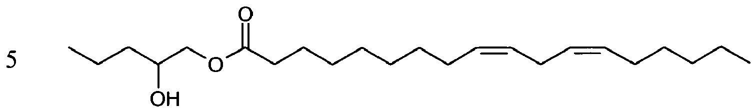

7. Composé de formule (I) selon l'une quelconque des revendications 1 à 6, pour son utilisation comme médicament.

8. Composé selon la revendication 7, **caractérisé en ce que** le médicament est destiné au traitement de l'acné ou de la dermite séborrhéique.

9. Composition pharmaceutique ou cosmétique comprenant au moins un composé de formule (I) selon l'une quelconque des revendications 1 à 6 en association avec au moins un excipient pharmaceutiquement ou cosmétiquement acceptable.

10. Composition pharmaceutique ou cosmétique selon la revendication 9, **caractérisée en ce qu'**elle comprend 0,01 à 10% en poids, de préférence 0,1% à 1% en poids, du ou des composés de formule (I) par rapport au poids total de la composition.

11. Méthode de traitement cosmétique de l'acné ou de la dermite séborrhéique comprenant l'application sur la peau d'une composition cosmétique selon la revendication 9 ou 10.

12. Procédé de préparation d'un composé de formule (I) selon l'une quelconque des revendications 1 à 6 par couplage d'un acide gras polyinsaturé choisi parmi les omégas 3 et les omégas 6, dont la fonction acide carboxylique se trouve sous forme libre ou activée, et d'un diol de formule (II) suivante : 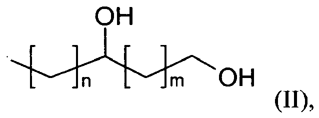 pour lequel n représente un nombre entier compris entre 1 et 15, et de préférence entre 1 et 10, et m représente 0, 1, 2 ou 3, et de préférence 0 ou 1.

13. Procédé selon la revendication 12, **caractérisé en ce que** la réaction de couplage est réalisée à partir de l'acide gras polyinsaturé, dont la fonction acide carboxylique se trouve sous forme libre, en présence d'un agent de couplage choisi dans le groupe constitué par le diisopropylcarbodiimide, le dicyclohexylcarbodiimide, le chlorhydrate de 1-(3-diméthylaminopropyl)-3-éthylcarbodiimide, le carbonyldiimidazole, le 2-H-benzotriazole-1-yl)-1,1,3,3-tetramethyluronium hexafluorophosphate, le 2-(1H-benzotriazole-1-yl)-1,1,3,3-tetramethyluronium tetrafluoroborate ou le O-(7-azobenzotriazol-1-yl)-1,1,3,3-tétraméthyluronium hexafluorophosphate, éventuellement associé à un auxiliaire de couplage choisi dans le groupe constitué par le N-hydroxy succinimide, le N-hydroxy benzotriazole, le 3,4-dihydro-3-hydroxy-4-oxo-1,2,3-benzotriazole, le 1-hydroxy-7-azabenzotriazole, la diméthylaminopyridine ou le N-hydroxysylfosuccinimide.

14. Procédé selon l'une quelconque des revendications 12 et 13, **caractérisé en ce que** l'agent de couplage est le chlorhydrate de 1-(3-diméthylaminopropyl)-3-éthylcarbodiimide et l'auxiliaire de couplage est la diméthylaminopyridine.

15. Procédé selon la revendication 12, **caractérisée en ce que** la réaction de couplage est réalisée à partir de l'acide gras polyinsaturé, dont la fonction acide carboxylique est activée sous forme d'un chlorure d'acide, notamment en présence de pyridine et de diméthylaminopyridine.

## Patentansprüche

1. Verbindung mit der nachfolgenden, allgemeinen Formel (I): 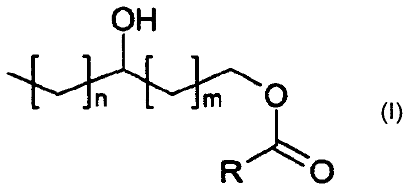 worin
- n eine ganze Zahl zwischen 1 und 15 darstellt,
- m 0, 1, 2 oder 3 darstellt, und
- R die Kohlenwasserstoffkette einer zwischen Omega-3 und Omega-6 gewählten, mehrfach ungesättigten Fettsäure darstellt.

2. Verbindung nach Anspruch 1, **dadurch gekennzeichnet, dass** n zwischen 1 und 10 liegt.

3. Verbindung nach einem der Ansprüche 1 und 2, **dadurch gekennzeichnet, dass** n 1, 2, 3, 4 oder 5 darstellt.

4. Verbindung nach einem der Ansprüche 1 bis 3, **dadurch gekennzeichnet, dass** m 0 oder 1 darstellt.

5. Verbindung nach einem der Ansprüche 1 bis 4, **dadurch gekennzeichnet, dass** die mehrfach ungesättigte Fettsäure ausgewählt ist aus α-Linolensäure, Stearidonsäure, Eicosatriensäure, Eicosatetraensäure, Eicosapentaensäure, Docosapentaensäure, Docosahexaensäure, Tetracosapentaensäure, Tetracosahexaensäure, Linolensäure, Gamma-Linolensäure, Eicosadiensäure, Dihomogammalinolensäure, Arachidonsäure, Docosatetraensäure, Docosapentaensäure, Adrensäure und Calendulasäure, vorzugsweise aus α-Linolensäure, Stearidonsäure oder Linolensäure.

6. Verbindung nach einem der Ansprüche 1 bis 5, **dadurch gekennzeichnet, dass** sie ausgewählt ist aus den nachfolgenden Molekülen:

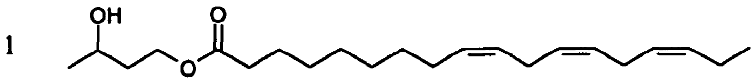

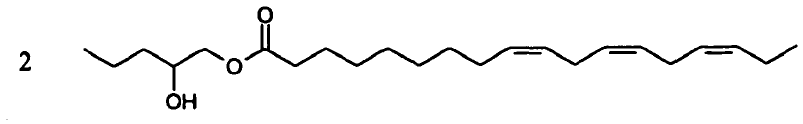

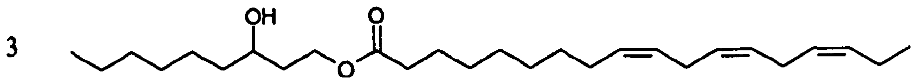

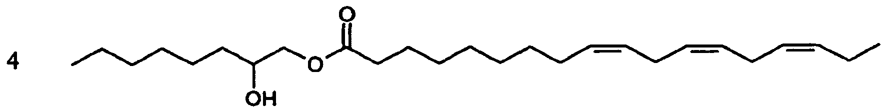

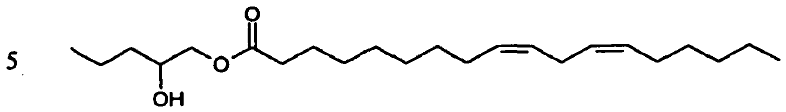

7. Verbindung der Formel (I) nach einem der Ansprüche 1 bis 6 zu deren Verwendung als Medikament.

8. Verbindung nach Anspruch 7, **dadurch gekennzeichnet, dass** das Medikament zur Behandlung von Akne oder seborrhoischer Dermitis bestimmt ist.

9. Pharmazeutische oder kosmetische Zusammensetzung mit zumindest einer Verbindung der Formel (I) nach einem der Ansprüche 1 bis 6 in Verbindung mit zumindest einem pharmazeutisch oder kosmetisch verträglichen Hilfsstoff.

10. Pharmazeutische oder kosmetische Zusammensetzung nach Anspruch 9, **dadurch gekennzeichnet, dass** sie 0,01 bis 10 Gew.-%, vorzugsweise 0,1 Gew.% bis 1 Gew.-% der Verbindung bzw. Verbindungen der Formel (I) in Bezug auf das Gesamtgewicht der Zusammensetzung enthält.

11. Verfahren zur kosmetischen Behandlung von Akne oder seborrhoischer Dermitis, umfassend die Anwendung einer kosmetischen Zusammensetzung nach Anspruch 9 oder 10 auf der Haut.

12. Verfahren zum Herstellen einer Verbindung der Formel (I) nach einem der Ansprüche 1 bis 6 durch Kopplung einer mehrfach ungesättigten Fettsäure, ausgewählt aus Omega-3 und Omega-6, deren Karbonsäure-Funktion in freier oder aktivierter Form vorliegt, und eines Diols der nachfolgenden Formel (II): 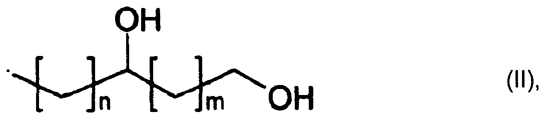 worin n eine ganze Zahl zwischen 1 und 15, vorzugsweise zwischen 1 und 10 darstellt, und m 0, 1, 2 oder 3, vorzugsweise 0 oder 1 darstellt.

13. Verfahren nach Anspruch 12, **dadurch gekennzeichnet, dass** die Kopplungsreaktion ausgehend von der mehrfach ungesättigten Fettsäure erfolgt, deren Karbonsäure-Funktion in freier Form vorliegt, und zwar bei einem vorhandenen Kopplungsmittel ausgewählt aus der Gruppe besteht aus Diisopropylcarbodiimid, Dicyclohexylcarbodiimid, 1-(3-Dimethylaminopropyl)-3-Ethylcarbodiimid-Hydrochlorid, Carbonyldiimidazol, 2-H-Benzotriazol-1-yl)-1,1,3,3,-Tetramethyluronium Hexafluorphosphat, 2-(IH-Benzotriazol-1-yl)-1,1,3,3-Tetrametyluronium Tetrafluorborat oder O-(7-Azobenzotriazol-1-yl)-1,1,3,3-Tetrametyhluronium Hexafluorphosphat, gegebenenfalls in Verbindung mit einem Kopplungshilfsmittel ausgewählt aus der Gruppe bestehend aus N-Hydroxysuccinimid, N-Hydroxybenzotriazol, 3,4-Dihydro-3-Hydroxy-4-oxo-1,2,3-Benzotriazol, 1-Hydroxy-7-Azabenzotriazol, Dimethylaminopyridin oder N-Hydroxysylfosuccinimid.

14. Verfahren nach einem der Ansprüche 12 und 13, **dadurch gekennzeichnet, dass** das Kopplungsmittel 1-(3-Dimethylaminopropyl)-3-Ethylcarbodiimid-Hydrochlorid und das Kopplungshilfsmittel Dimethylaminopyridin ist.

15. Verfahren nach Anspruch 12, **dadurch gekennzeichnet, dass** die Kopplungsreaktion ausgehend von der mehrfach ungesättigten Fettsäure erfolgt, deren Karbonsäure-Funktion in Form von Säurechlorid aktiviert wird, insbesondere bei vorhandenem Pyridin und Dimetylaminopyridin.

## Claims

1. A compound of the following general formula (I): 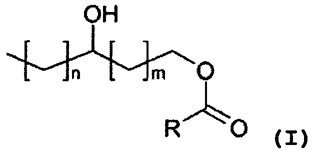 wherein:
- n is an integer between 1 and 15,
- m is 0, 1, 2 or 3, and
- R is the hydrocarbon chain of a polyunsaturated fatty acid selected from omega-3 and omega-6 polyunsaturated fatty acids.

2. The compound according to claim 1, **characterized in that** n is between 1 and 10.

3. The compound according to either of claims 1 and 2, **characterized in that** n is 1, 2, 3, 4 or 5.

4. The compound according to any of claims 1 to 3, **characterized in that** m is 0 or 1.

5. The compound according to any of claims 1 to 4, **characterized in that** the polyunsaturated fatty acid is selected from α-linolenic acid, stearidonic acid, eicosatrienoic acid, eicosatetraenoic acid, eicosapentaenoic acid, docosapentaenoic acid, docosahexaenoic acid, tetracosapentaenoic acid, tetracosahexaenoic acid, linoleic acid, γ-linolenic acid, eicosadienoic acid, dihomo-γ-linolenic acid, arachidonic acid, docosatetraenoic acid, docosapentaenoic acid, adrenic acid and calendic acid, and preferably it is α-linolenic acid, stearidonic acid or linoleic acid.

6. The compound according to any of claims 1 to 5, **characterized in that** it is selected from the following molecules:

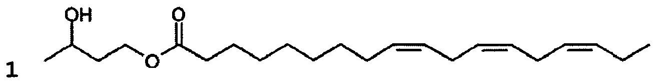

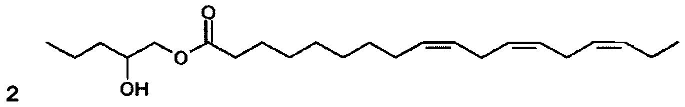

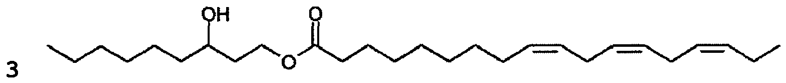

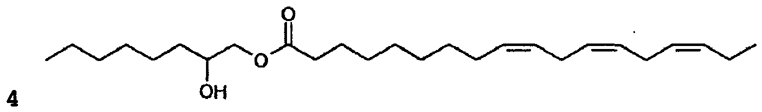

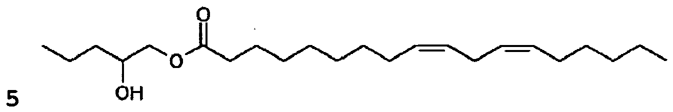

7. The compound of the formula (I) according to any of claims 1 to 6, for its use as a drug.

8. The compound according to claim 7, **characterized in that** the drug is intended to treat acne or seborrheic dermatitis.

9. A pharmaceutical or cosmetic composition comprising at least one compound of the formula (I) according to any of claims 1 to 6 in combination with at least one pharmaceutically or cosmetically acceptable excipient.

10. The pharmaceutical or cosmetic composition according to claim 9, **characterized in that** said composition comprises 0.01% to 10% by weight, preferably 0.1% to 1% by weight, of the said one or more compounds of the formula (I) compared to the total weight of the composition.

11. A method for the cosmetic treatment of acne or seborrheic dermatitis comprising the application on the skin of a cosmetic composition according to claim 9 or 10.

12. A method for preparing a compound of the formula (I) according to any of claims 1 to 6 by coupling a polyunsaturated fatty acid selected from omega-3 and omega-6 fatty acids, whose carboxylic acid function is in free or activated form, and a diol of the following formula (II): 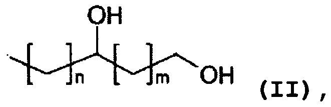 wherein n is an integer between 1 and 15, preferably between 1 and 10, and m is 0, 1, 2 or 3, preferably 0 or 1.

13. The method according to claim 12, **characterized in that** the coupling reaction is carried out from the polyunsaturated fatty acid, whose carboxylic acid function is in free form, in the presence of a coupling agent selected from the group consisting of diisopropylcarbodiimide, dicyclohexylcarbodiimide, 1-(3-dimethylaminopropyl)-3-ethylcarbodiimide hydrochloride, carbonyldiimidazole, 2-H-benzotriazole-1-yl)-1,1,3,3-tetramethyluronium hexafluorophosphate, 2-(1H-benzotriazole-1-yl)-1,1,3,3-tetramethyluronium tetrafluoroborate or O-(7-azobenzotriazol-1-yl)-1,1,3,3-tetramethyluronium hexafluorophosphate, optionally combined with a coupling auxiliary selected from the group consisting of N-hydroxysuccinimide, N-hydroxybenzotriazole, 3,4-dihydro-3-hydroxy-4-oxo-1,2,3-benzotriazole, 1-hydroxy-7-azabenzotriazole, dimethylaminopyridine or N-hydroxysulfosuccinimide.

14. The method according to either of claims 12 and 13, **characterized in that** the coupling agent is 1-(3-dimethylaminopropyl)-3-ethylcarbodiimide hydrochloride and the coupling auxiliary is dimethylaminopyridine.

15. The method according to claim 12, **characterized in that** the coupling reaction is carried out from the polyunsaturated fatty acid, whose carboxylic acid function is activated in the form of an acid chloride, in particular in the presence of pyridine and dimethylaminopyridine.
